(19) Europäisches Patentamt
European Patent Office
Office européen des brevets

(11) **EP 4 467 189 A1**

(12) **EUROPEAN PATENT APPLICATION**
published in accordance with Art. 153(4) EPC

(43) Date of publication:
**27.11.2024 Bulletin 2024/48**

(21) Application number: **23740263.1**

(22) Date of filing: **11.01.2023**

(51) International Patent Classification (IPC):
**A61N 2/02** (2006.01)

(52) Cooperative Patent Classification (CPC):
**A61N 2/02**

(86) International application number:
**PCT/JP2023/000402**

(87) International publication number:
**WO 2023/136254 (20.07.2023 Gazette 2023/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.01.2022 JP 2022004902**

(71) Applicants:
• **Nipro Corporation
Settsu-shi, Osaka 566-8510 (JP)**
• **Harada Electronics Industry Co., Ltd.
Sapporo-shi, Hokkaido 063-0052 (JP)**
(72) Inventors:
• **MURAKAMI Masaki
Yamanashi 4093801 (JP)**
• **NAKAZAWA Koji
Yamanashi 4093801 (JP)**
• **HARADA Masahide
Sapporo-shi, Hokkaido 063-0052 (JP)**

(74) Representative: **Mewburn Ellis LLP
Aurora Building
Counterslip
Bristol BS1 6BX (GB)**

(54) **MAGNETIC THERAPY DEVICE**

(57)    Provided is a magnetic treatment apparatus that has a high magnetic treatment effect and is capable of properly conforming with an affected area. The magnetic treatment apparatus is an apparatus for treating pain in an affected area of a living body by producing a biostimulation signal wave and stimulating the cells and nerves in and around the affected area as a result of irradiating the affected area with a magnetic field for the affected area stimulation that is generated at a coil via the biostimulation signal wave, including: an apparatus main body having a signal wave output part configured to produce and output a biostimulation high-frequency signal; and a probe formed separately from the apparatus main body and having a coil for high-frequency output that is connected to the signal wave output part through a signal cable and is supplied with the biostimulation high-frequency signal output from the signal wave output part; wherein a high-frequency current of 100 MHz or higher is applied to the coil for high-frequency output, the coil for high-frequency output is formed as a planar body on both surfaces of an insulating flexible sheet, and centers of magnetic fields generated by the coils on both surfaces are coincident with each other.

Fig. 4

EP 4 467 189 A1

## Description

Technical field

The present invention relates to an apparatus for treating pain in an affected area of a living body by producing a biostimulation signal wave and stimulating the cells and nerves in the affected area as a result of irradiating the affected area with a magnetic field that is generated at a coil via such signal wave.

Background Art

As an apparatus for treating pain in an affected area of a living body by stimulating the cells and nerves in the affected area as a result of irradiating the affected area with a magnetic field, there is conventionally known, for example, an apparatus disclosed in Patent Literature 1. This treatment apparatus is configured in such a manner that a helical high-frequency coil and a helical low-frequency coil are aligned together in a housing, or a looped high-frequency coil and a looped low-frequency coil are stacked together in a housing, whereby these coils are received in the housing along with an oscillation circuit and a battery, which allows the apparatus to be carried around.

Further, this treatment apparatus treats pain in an affected area in the following manner. That is, magnetic fields are respectively generated at these high-frequency coil and low-frequency coil via a high-frequency signal and a low-frequency signal with certain frequencies that are output from the oscillation circuit. By placing the housing on an affected area of a living body, the affected area will be irradiated with the magnetic fields to have the cells and nerves in the affected area stimulated, whereby this stimulation activates the self-repair function of the affected area to repair the cells and tissues therein.

As opposed to magnetic treatment devices employing static magnetic fields with the use of permanent magnets, magnetic treatment apparatuses relieving muscle fatigue or the like via magnetic fields that are generated from coils by applying high-frequency currents to the coils employ dynamic magnetic fields with high frequencies and are thus said to have higher effects due to a lesser degree of accustomization of the body.

Further, a magnetic treatment apparatus is required to have a compact size and a flexible deformability as the apparatus is used in such a way that it needs to conform with an affected area of the body. Particularly, in the case of a magnetic treatment apparatus requiring a high-frequency current generation unit, it is critical to realize downsizing and flexibility.

Meanwhile, in order to generate an intense magnetic field, either a large current has to be applied, or the number of turns in a coil has to be increased.

For example, disclosed in Patent Literature 2 is a high-frequency magnetic treatment device in which a coil is formed on one surface of a flexible printed board, and a high-frequency oscillation integrated circuit part for supplying a high-frequency current to the coil is provided on the printed board.

In addition, disclosed in Patent Literature 3 is a high-frequency magnetic treatment device in which the number of turns in a coil is increased, a coil part is housed in a casing, and the casing is made of a paramagnetic material except for a surface thereof that comes into contact with the body.

Citation List

Patent Literature

Patent Literature 1: WO2008/056414
Patent Literature 2: JP-A-S63(1988)-160676
Patent Literature 3: JP-A-S63(1988)-160677

Summary of Invention

Technical Problem

However, the problem with the apparatus disclosed in Patent Literature 1 is that since the high-frequency coil and the low-frequency coil are received in the housing together with the oscillation circuit and the battery, the housing needs to be placed on an affected area in order for the affected area to be irradiated with the magnetic fields generated at these coils, thereby making the housing bulky and a hindrance, or even causing the apparatus to fall off the affected area after being caught on clothes.

In this regard, it was made clear that if downsizing the housing in order to solve this problem, the oscillation circuit and the battery need to be downsized as well, which will incur another problem that a sufficient magnetic field and operating time may not be achieved.

The problem with the technique disclosed in Patent Literature 2 is that a coil with a plural number of turns is formed on one surface of the flexible printed board, and the back surface of the printed board is only used for lead wires, which fails to make effective use of the back surface. Since the number of turns in the coil is plural, there is a problem of heat generation due to electrical resistance if applying a large current.

The problem with the technique disclosed in Patent Literature 3 is that since the coil is received in a rigid case such as one made of aluminum or iron in order to resolve the disorder of high-frequency leakage electromagnetic waves that is incurred as a result of increasing the number of turns in the coil, the device cannot conform with an affected area in a flexible manner.

The present invention was made in view of the above circumstances, and it is an object of the present invention to provide a magnetic treatment apparatus that has a high magnetic treatment effect and is capable of properly conforming with an affected area.

Solution to Problem

The magnetic treatment apparatus according to the present invention, which favorably solves the above problems, is a magnetic treatment apparatus for treating pain in an affected area of a living body by producing a biostimulation signal wave and stimulating the cells and nerves in and around the affected area as a result of irradiating the affected area of the living body with a magnetic field for stimulating the affected area that is generated at a coil via the biostimulation signal wave, and comprises: an apparatus main body having a signal wave output part configured to produce and output a biostimulation high-frequency signal; and a probe formed separately from the apparatus main body and having a coil for high-frequency output that is connected to the signal wave output part through a signal cable and is supplied with the biostimulation high-frequency signal output from the signal wave output part, in which a high-frequency current of 100 MHz or higher is applied to the coil for high-frequency output, the coil for high-frequency output is formed as a planar body on both surfaces of an insulating flexible sheet, and centers of magnetic fields generated by the coils on both surfaces are coincident with each other.

Advantageous Effects of Invention

In the magnetic treatment apparatus according to the present invention, the signal wave output part in the apparatus main body produces and outputs the biostimulation high-frequency signal, the probe formed separately from the apparatus main body has the coil for high-frequency output, the coil is connected to the signal wave output part through the signal cable and is supplied with the biostimulation high-frequency signal output from the signal wave output part, thereby allowing the coil for high-frequency output to generate a high-frequency alternating magnetic field for affected area stimulation with such biostimulation high-frequency signal.

Thus, according to the magnetic treatment apparatus of the present invention, by placing the probe separated from the apparatus main body on an affected area of a living body, the affected area will be irradiated with the high-frequency alternating magnetic field generated at the coil for high-frequency output to have the cells and nerves in the affected area stimulated, and this stimulation will activate, for example, the damaged nerves in the affected area to bring an expectation that the nerve disorder in the affected area can be alleviated via self-repairing.

In addition, according to the magnetic treatment apparatus of the present invention, a high-frequency current of 100 MHz or higher is applied to the coil for high-frequency output, the coil for high-frequency output is formed as a planar body on both surfaces of the insulating flexible sheet, and the centers of the magnetic fields generated by the coils on both surfaces are coincident with each other. Therefore, the probe is allowed to properly conform with an affected area, and the affected area can be irradiated with an intense high-frequency alternating magnetic field to have the cells and nerves in the affected area stimulated. This stimulation promotes, for example, the production of the neurotrophic factors in the cells of the affected area, thereby bringing an expectation that the pain in the affected area can be treated as a result of facilitating the repair, growth, differentiation, and proliferation of these cells.

Incidentally, in the magnetic treatment apparatus according to the present invention, it is preferable if the coil for high-frequency output is made of a conductor having a thickness that is not smaller than twice a current skin depth $d_i$ calculated from the frequency of the high-frequency current and is not larger than 70 $\mu$m, because there can be realized both a flexibility of the probe and prevention of heat generation due to a reduction in electrical resistance of the coil for high-frequency output. Further, it is preferable if the conductor is a metal foil, because the apparatus of the present invention can be produced economically.

Further, in the magnetic treatment apparatus according to the present invention, the signal wave output part may also produce and output a biostimulation low-frequency signal, and the probe may also have a coil for low-frequency output that is connected to the signal wave output part through the signal cable and is supplied with the biostimulation low-frequency signal output from the signal wave output part. In this way, the stimulus delivered on an affected area as a result of irradiating the affected area with a low-frequency alternating magnetic field for affected area stimulation that is generated at the coil for low-frequency output via the biostimulation low-frequency signal will reach the brain (sensory area) from the spinal cord dorsal horn through sensory nerves (Aβ fibers: sense of touch), thereby allowing the brain to recognize a pleasant sense of touch, and thus also bringing an expectation that a pain-relieving effect and a relaxing effect can be achieved by activating the descending pain inhibitory system.

Brief Description of Drawings

[FIG. 1] Fig. 1 is an overall conceptual diagram showing a magnetic treatment apparatus of an embodiment according to the present invention.
[FIG.2] Figs. 2(a) to (c) are a set of schematic diagrams showing a probe of the magnetic treatment apparatus of the above embodiment, in which Fig. 2(a) is a top view, Fig. 2(b) is a back view, and Fig. 2(c) is an A-A cross-sectional view.
[FIG.3] Figs. 3(a), (b) are a set of printed wiring diagrams showing a coil arrangement of the magnetic treatment apparatus of the above embodiment, in which Fig. 3(a) is a top view, and Fig. 3(b) is a bottom view.
[FIG.4] is a B-B cross-sectional view of Fig. 3(a), illustrating a skin effect caused by a high-frequency current.

Description of Embodiments

An embodiment according to the present invention will be described in detail hereunder based on the drawings. Here, the accompanying drawings are schematic and may differ from the embodiment in reality. Further, the following embodiment is a set of examples of an apparatus embodying the technical concept of the present invention and is not to limit the configuration of the present invention to those shown below. That is, various modifications can be made to the technical concept of the present invention within the technical scope described in the claims.

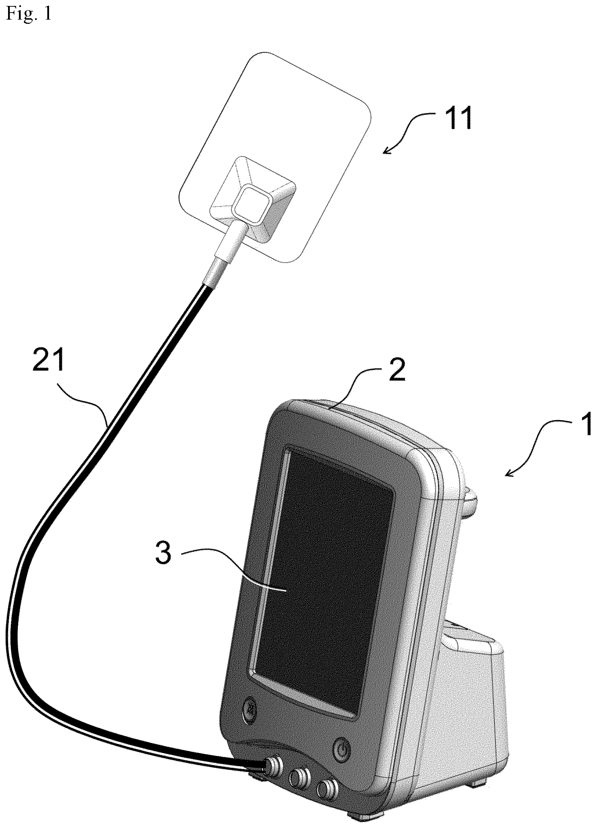

Fig. 1 is a perspective view showing an overall concept of a magnetic treatment apparatus of an embodiment according to the present invention. In the subsequent drawings, a reference sign "1" represents an apparatus main body of the magnetic treatment apparatus of this embodiment, a reference sign "11" represents a probe, and a reference sign "21" represents a signal cable. Further, the magnetic treatment apparatus has a power cable (not shown) that can be detachably plugged into and attached to the apparatus main body 1.
The apparatus main body 1 of the magnetic treatment apparatus of this embodiment has a resin casing 2; a touch panel-type display 3 that is housed obliquely upward in a front portion inside the casing 2 and is exposed from an opening section of a front surface of the casing 2; a signal wave output part that is housed in a rear upper portion inside the casing 2; and a power supply part that is housed in a rear lower portion inside the casing 2. Here, an alarm-stop button and a power switch button are provided on the left and the right side below the opening section on the front surface of the casing 2 of the apparatus main body 1. Moreover, provided even below these buttons are three horizontally aligned sockets for plugging in the signal cable 21 so that three probes 11 can be connected to the apparatus main body 1.

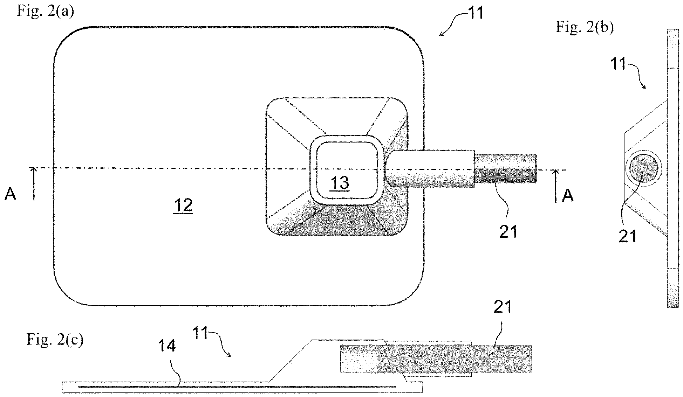

Figs. 2 include a top view Fig. 2(a), a back view Fig. 2(b), and a A-A cross-sectional view Fig. 2(c) of the probe 11 of the magnetic treatment apparatus of this embodiment. In this embodiment, the probe 11 has a substantially rectangular outer package 12 made of a flexible material; and a circuit part 13 bulged into a truncated pyramid shape for the purpose of protecting electric circuits or the like. The signal cable 21 is drawn from the back side of the circuit part.
As shown in Fig. 2(c), the probe 11 is configured in such a manner where a flexible sheet 14 consisted of a printed board with coils and electric circuits arranged thereon is housed in the outer package 12 that is made of a soft resin such as an elastomer and a rubber material. The probe 11 can be produced via resin molding such as injection molding and RIM molding. Since resin molding involves self-heating, it is preferred that resin molding be performed on the circuit part 13 in advance for the purpose of protecting electric circuits or the like.

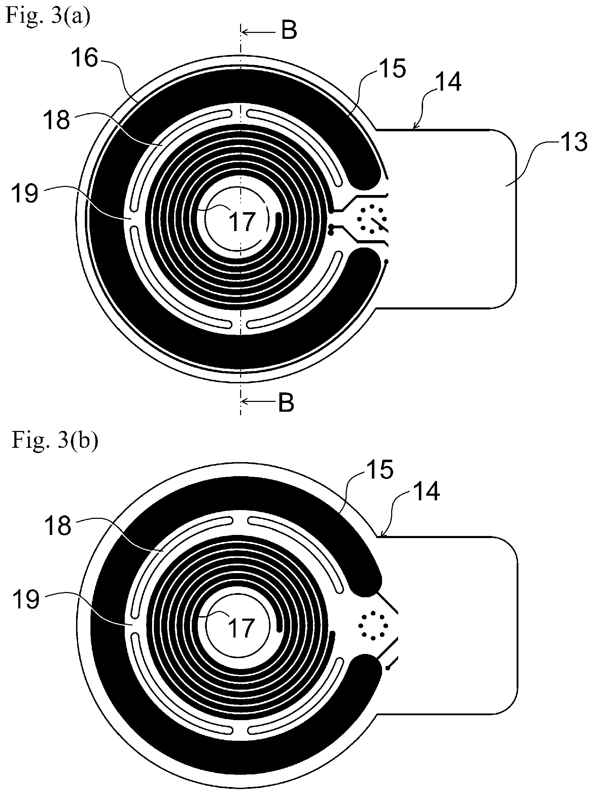

Figs. 3(a) and 3(b) are a set of printed wiring diagrams showing a coil arrangement of the magnetic treatment apparatus of this embodiment, where Fig. 3(a) is a top view, and Fig. 3(b) is a bottom view. Arranged on the printed board 14 are the circuit part 13, a coil 15 for high-frequency output, a coil 16 for magnetic field intensity detection, a coil 17 for low-frequency output and the like. In this embodiment, the coil 15 for high-frequency output and the coil 16 for magnetic field intensity detection are arranged on the upper surface of the printed board 14 in such a manner that the coil 15 for high-frequency output is provided as an annular disk, and the coil 16 for magnetic field intensity detection that has an annular shape is provided outside such coil 15 for high-frequency output. Further, the coil 17 for low-frequency output that has a spiral shape may be arranged inside the coil 15 for high-frequency output. The coil 15 for high-frequency output and the coil 17 for low-frequency output are also arranged on the lower surface of the printed board 14, whereby a plane-symmetrical configuration is established so that a high-frequency current and a low-frequency current can respectively flow in the same direction on both the upper and lower surfaces. In this way, the centers of the magnetic fields generated by the coils on both the upper and lower surfaces can be coincident with each other. Slit sections 18 are provided between the coil 15 for high-frequency output and coil 17 for low-frequency output of the printed board 14, whereby a followability of the printed board 14 is improved upon deformation of the probe 11. Further, multiple parts on the circumference of the slit sections 18 are connected to one another through bridge portions 19, which makes it possible to stably carry out molding when covering the printed board via resin molding. Incidentally, instead of an annular disk, the coil 15 for high-frequency output may also be formed into a rectangular shape.

The flexible sheet 14 is consisted of a film-shaped printed board preferably having a base layer that is composed of a film made of a bendable insulating material; and, if necessary, an adhesive layer. The base layer may employ, for example, a polyimide resin, a polyester resin, a polyamide paper substrate epoxy resin, a glass cloth substrate epoxy resin, and a glass substrate BT resin. Each coil may be a conductor printed or etched on the printed board 14, such as a metal foil. The printed board 14 preferably has a thickness of about 0.1 mm.

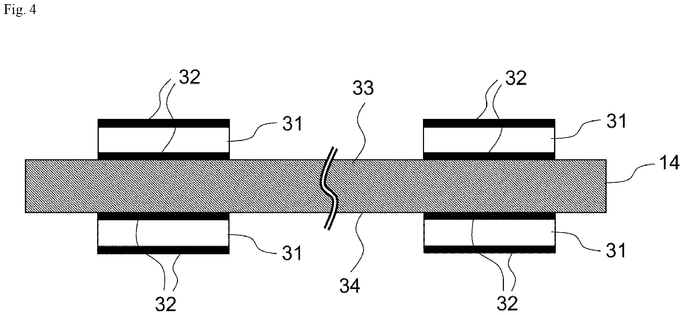

Fig. 4 shows a B-B cross-sectional view of Fig. 3(a). The coil 15 for high-frequency output of this embodiment is configured as, for example, a copper foil 31 on both an upper surface 33 and lower surface 34 of the printed board 14. The high-frequency current intensely flows in a skin portion 32 of the conductor due to a so-called skin effect. As shown in Figs. 3 and 4, the coils 15 for high-frequency output are arranged on both the upper and lower surfaces with the centers of their magnetic fields being coincident with each other, thereby increasing the surface area of the conductor in which the high-frequency current flows, thus contributing to downsizing the probe 11. A skin depth $d_i$ involving a high-frequency current is calculated by the following formula 1. In the formula 1, $\rho$ represents the electrical resistivity of the conductor; $\omega$ represents the angular frequency of the current; $\mu$ represents the absolute permeability of the conductor.

[Formula 1]

$$d_i = \sqrt{\frac{2\rho}{\omega\mu}} \qquad\qquad (1)$$

In the case of copper at normal temperature, the skin depth $d_i$ involving a high-frequency current with a frequency of 100 MHz is calculated to be about 6 $\mu$m based on the above formula 1. If the thickness of the coil 15 for high-frequency output is smaller than twice the skin depth $d_i$, the coil may generate an excessively large amount of heat due to electrical resistance; meanwhile, if the thickness of the coil 15 for high-frequency output is larger than 70 $\mu$m, a followability upon deformation of the probe 11 may be lost, which may cause the coil to be peeled away from the printed board or cause disconnection.

The frequency of the high-frequency current applied to the coil for high-frequency output is about 100 MHz to 400 MHz, and the frequency is changed continuously or at given time intervals to prevent the affected area from being accustomed to the stimulus signal. The frequency of the high-frequency current is preferably 200 MHz to 300 MHz.

The frequency of the low-frequency current applied to the coil for low-frequency output is about 1 kHz to 3 kHz. It is preferred that the center of the magnetic field generated by the coil for high-frequency output and the center of the magnetic field generated by the coil for low-frequency output are coincident with each other. This allows a low-frequency magnetic field to be supplied to the affected area in addition to the high-frequency magnetic field in a superimposed manner, thereby achieving a higher magnetic treatment effect.

In the magnetic treatment apparatus of this embodiment, the apparatus main body 1, from a functional perspective, has the signal wave output part, a screen control part, and the power supply part. The circuit configuration of the signal wave output part in this embodiment is such that it is configured using a plurality of central processing units (CPU). In the signal wave output part, a basic high-frequency signal of 100 MHz or higher is generated at a basic high-frequency signal generation part, and this basic high-frequency signal is then processed by a basic high-frequency signal shifting part in such a manner where, for example, with 250 MHz being the center frequency, and within a range of 225 MHz to 275 MHz which is ±10% from such center frequency, the basic high-frequency signal is moderately shifted (varied), for example, every 0.00014 seconds (i.e., about 7,000 times/second) as a fixed amount of time while excluding the frequency band used in emergency locator transmitter for an airplane. The basic high-frequency signal thus shifted is then output to a basic high-frequency signal frequency modulation part.

The signal wave output part is also configured as follows. A magnetic signal pattern read-out part reads out magnetic signal patterns including sound source signals such as music from, for example, a built-in storage device or an external storage device such as an SD card inserted in a card slot or the like, the magnetic signal patterns being previously recorded in such built-in or external storage device. The magnetic signal patterns are then supplied to a biostimulation low-frequency signal generation part, whereby a biostimulation low-frequency signal is generated by the biostimulation low-frequency signal generation part based on the frequency information (e.g., 1 kHz to 3 kHz) of the magnetic signal patterns, and the biostimulation low-frequency signal is then output to the basic high-frequency signal frequency modulation part.

Further, using a biostimulation low-frequency signal of, for example, 1 kHz to 3 kHz that has been generated at the biostimulation low-frequency signal generation part, the basic high-frequency signal frequency modulation part modulates the frequency of a basic high-frequency signal of, for example, 250 MHz $\pm 10\%$ that has been generated at the basic high-frequency signal generation part and then has its frequency shifted at the basic high-frequency signal shifting part. The frequency-modulated signal is then supplied to a biostimulation high-frequency signal output part, whereby the biostimulation high-frequency signal output part amplifies the frequency-modulated biostimulation high-frequency signal and then outputs the signal. Incidentally, the biostimulation high-frequency signal output part may modulate the amplitude of the frequency-modulated biostimulation high-frequency signal with the aforementioned biostimulation low-frequency signal and then amplifying and outputting the biostimulation high-frequency signal. Further, a biostimulation low-frequency signal output part may amplify, for example, a biostimulation low-frequency signal of 1 kHz to 3 kHz that has been generated at the biostimulation low-frequency signal generation part, and then output the signal. These operations in the signal wave output part are controlled by an operation state control part.

The probe 11 of this embodiment is such that the flexible printed-wiring board is housed therein, and formed via printed wiring on such flexible printed-wiring board are the coil 15 for high-frequency output that is arranged on the outer side and the coil 17 for low-frequency output that is arranged on the inner side. In addition, the coil 16 for magnetic field intensity detection is formed outside the coil 15 for high-frequency output. Moreover, the circuit configuration of an operation state detection part is such that it is configured using a temperature detection element and a CPU that are mounted on such flexible printed-wiring board. The coil 15 for high-frequency output generates a high-frequency alternating magnetic field for affected area stimulation with the biostimulation high-frequency signal that is supplied from the biostimulation high-frequency signal output part through the signal cable 21. The coil 17 for low-frequency output may generate a low-frequency alternating magnetic field for affected area stimulation with the biostimulation low-frequency signal that is supplied from the biostimulation low-frequency signal output part through the signal cable 21.

In the meantime, the operation state detection part detects the operation state of the signal wave output part from the temperatures of the coil 15 for high-frequency output and the coil 17 for low-frequency output that are detected by the temperature detection element, and from the high-frequency or low-frequency magnetic field intensity that is detected by the coil 16 for magnetic field intensity detection. The operation state detection part then inputs a signal indicating such operation state into an operation state monitoring part of the signal wave output part through the signal cable 21. With this signal indicating the operation state, the operation state monitoring part is able to monitor operations such as the generation and output of signal waves by the signal wave output part as well as the levels of the alternating magnetic fields generated by the coil 15 for high-frequency output and the coil 17 for low-frequency output, and output an alarm signal (e.g., sounding of an alarm by a speaker that is built in the apparatus main body 1 and is not shown, display of an alarm message on the display 3 etc.) via an alarm control part if detecting an abnormality. Incidentally, the sounding of the alarm is stopped by operating the alarm stop button provided on the front surface of the casing of the apparatus main body 1. Further, for the sake of ensuring the safety of the user of this magnetic treatment apparatus, once an abnormality has been detected, the operation state control part will immediately stop the supply of the biostimulation high-frequency signal from the high-frequency signal output part to the coil 15 for high-frequency output, and the supply of the biostimulation low-frequency signal from the biostimulation low-frequency signal output part to the coil 17 for low-frequency output.

The circuit configuration of the screen control part in this embodiment is such that it is configured using a graphics processing unit (GPU) or the like. With an image display part, screen information such as instruction buttons that are to be displayed on a liquid crystal display (LCD) or the like of the touch input-type display 3 is read out from an SD card or the like and then displayed on such LCD or the like, the screen information being previously recorded in an external storage device such as an SD card inserted in a card slot or the like. Further, with an instruction input part, a position at which a finger of the magnetic treatment apparatus's user has touched the touch panel of the touch input-type display 3 is detected based on, for example, changes in static electricity at such position, and a signal indicating the instruction input directed by the operation button displayed on the LCD or the like in response to such touch position is then sent to the operation state control part. This signal indicating an instruction input allows the operation state control part to control, for example, operations such as the generation (i.e. production) and output of signal waves by the signal wave output part as well as the alternating magnetic fields generated by the coil 15 for high-frequency output and the coil 17 for low-frequency output, in accordance with the user's instruction(s).

The screen control part further has a clock function where there is created a log storing the instruction inputs to the instruction input part as directed by the operation buttons displayed on the LCD or the like and the corresponding operation states of the signal wave output part, the information of this log is to be stored in a USB memory that is not shown and is installed in a USB memory slot provided in a protruding part on the rear lower portion of the casing 2 in such a manner that the USB memory can be inserted into and removed from the protruding part from an upper surface side thereof, and a clock is displayed on the LCD or the like at the image display part. This clock function is maintained by button cells placed in a battery holder.

The circuit configuration of the power supply part in this embodiment is such that it is configured by a CPU and two AC-DC converters that are not shown. Particularly, a commercial alternating-current power source of 100V that is supplied through a power cable (not shown) is subjected to the two AC-DC converters to obtain given direct-current power sources. Here, not only a battery can be charged as a charging voltage for the battery is achieved by connecting these given direct-current power sources in series, but also these given direct-current power sources are turned with switching power sources into other given direct-current power sources and then the other given direct-current power sources are stepped down by linear regulators before being supplied to the signal wave output part and screen control part of the apparatus main body 1 and the operation state detection part of the probe 11 as direct-current power sources with voltages required for each part.

Further, during a normal use of the magnetic treatment apparatus to which the power cable is not attached, the power supply part supplies the direct-current power sources from the battery to the signal wave output part and screen control part of the apparatus main body 1 and the operation state detection part of the probe 11 in such a manner that the direct-current power sources are turned with the switching power sources and the linear regulators to have their voltages stepped down to direct-current voltages required for each part, thereby allowing this magnetic treatment apparatus to be used in a portable manner.

In the magnetic treatment apparatus of this embodiment, the biostimulation high-frequency signal is generated (i.e. produced) by the basic high-frequency signal generation part, basic high-frequency signal shifting part, and basic high-frequency signal frequency modulation part of the signal wave output part in the apparatus main body 1, and the biostimulation high-frequency signal is then output by the biostimulation high-frequency signal output part. Further, the coil 15 for high-frequency output in the probe 11 formed separately from the apparatus main body 1 is connected to the biostimulation high-frequency signal output part of the signal wave output part through the signal cable 21 and is supplied with a biostimulation high-frequency signal that is output from such biostimulation high-frequency signal output part and has a center frequency of, for example, 250 MHz, thereby generating a high-frequency alternating magnetic field for affected area stimulation with such biostimulation high-frequency signal.

Thus, according to the magnetic treatment apparatus of this embodiment, by placing the probe 11 separate from the apparatus main body 1 on an affected area of a living body, the high-frequency alternating magnetic field generated by the coil 15 for high-frequency output will irradiate the affected area to stimulate the cells and nerves in the affected area, whereby, for example, the damaged sensory cells in the affected area are activated by such stimulus to induce neurotrophic factors, which brings an expectation that the nerve disorder in the affected area can be alleviated, and an expectation that the effect of alleviating the nerve disorder in the affected area can be enhanced since the high-frequency alternating magnetic field of 250 MHz at the center has a high effect of inducing neurotrophic factors by activating the damaged sensory cells.

Further, according to the magnetic treatment apparatus of this embodiment, the biostimulation low-frequency signal is generated by the biostimulation low-frequency signal generation part of the signal wave output part in the apparatus main body 1 based on the frequency information (e.g., 1 kHz to 3 kHz) of the magnetic signal patterns, and such biostimulation low-frequency signal is then output by the biostimulation low-frequency signal output part. The coil 17 for low-frequency output in the probe 11 is connected to the biostimulation low-frequency signal output part of the signal wave output part through the signal cable 21 and is supplied with the biostimulation low-frequency signal from such biostimulation low-frequency signal output part. In this way, the stimulus delivered on an affected area as a result of irradiating the affected area with the low-frequency alternating magnetic field for affected area stimulation that is generated at the coil 17 for low-frequency output via such biostimulation low-frequency signal will reach the brain (sensory area) from the spinal cord dorsal horn through sensory nerves (Aβ fibers: sense of touch), thereby allowing the brain to recognize a pleasant sense of touch, and thus also bringing an expectation that a pain-relieving effect and a relaxing effect can be achieved by activating the descending pain inhibitory system.

Moreover, according to the magnetic treatment apparatus of this embodiment, the basic high-frequency signal modulation part of the signal wave output part 13 in the apparatus main body 1 generates the biostimulation high-frequency signal by modulating the frequency of the basic high-frequency signal with the biostimulation low-frequency signal generated by the signal wave output part, and the biostimulation high-frequency signal output part supplies such frequency-modulated biostimulation high-frequency signal to the coil 15 for high-frequency output of the probe 11. In this way, by stimulating the cells and nerves in the affected area with the high-frequency alternating magnetic field for affected area stimulation that is generated at the coil 15 for high-frequency output via the biostimulation high-frequency signal that has been frequency-modulated with the biostimulation low-frequency signal, the damaged sensory cells in the affected area are more activated as compared to when no frequency modulation is conducted, thereby inducing more neurotrophic factors, thus bringing an expectation that the nerve disorder in the affected area can be more alleviated.

In addition, according to the magnetic treatment apparatus of this embodiment, the signal wave output part of the apparatus main body 1 also generates and outputs the biostimulation low-frequency signal, the probe 11 also has the coil 17 for low-frequency output that is connected to the signal wave output part through the signal cable 21 and is supplied with the biostimulation low-frequency signal from such signal wave output part, and the signal wave output part generates the biostimulation high-frequency signal by modulating the frequency of the basic high-frequency signal with the biostimulation low-frequency signal and then outputs such biostimulation high-frequency signal separately from the biostimulation low-frequency signal. In this way, not only a pain-relieving effect and a relaxing effect can be brought about by the low-frequency alternating magnetic field for affected area stimulation that is generated at the coil 17 for low-frequency output via the biostimulation low-frequency signal, but there is also an expectation that the nerve disorder in the affected area can be more alleviated with the high-frequency alternating magnetic field for affected area stimulation that is generated at the coil 15 for high-frequency output via the biostimulation high-frequency signal obtained by modulating the frequency of the basic high-frequency signal with the biostimulation low-frequency signal.

Further, according to the magnetic treatment apparatus of this embodiment, the frequency of the biostimulation low-frequency signal is not lower than 1 kHz and not higher than 3 kHz, and the stimulus delivered by the low-frequency alternating magnetic field of 1 kHz to 3 kHz that is generated at the coil 17 for low-frequency output via such biostimulation low-frequency signal is particularly easy to be transmitted from the spinal cord dorsal horn to the brain through sensory nerves, thereby bringing an expectation that there can be achieved nerve system-related effects such as a higher pain-relieving effect and relaxing effect.

The embodiment shown in the drawings has been described as above. However, the magnetic treatment apparatus according to the present invention shall not be limited to the above embodiment and may be appropriately modified within the scope of the descriptions in the claims; for example, the basic high-frequency signal shifting part may be configured to shift the basic high-frequency signal of 250 MHz within a range of, for example, 250 MHz $\pm$20%.

Moreover, for example, the biostimulation low-frequency signal generation part may be configured to generate a biostimulation low-frequency signal having a frequency range of, for example, 200 Hz to 3 kHz with 1.6 kHz being the center frequency, other than the frequency range of 1 kHz to 3 kHz.

Industrial Applicability

The magnetic treatment apparatus according to the present invention is industrially useful because with its downsized probe, the cells and nerves in an affected area of a living body can be stimulated when irradiated properly with the magnetic fields that are generated at the coils via the biostimulation signal waves generated, and this stimulation activates, for example, the self-repair function of the damaged nerves and cells in the affected area to bring an expectation that the nerve disorder in the affected area can be alleviated.

Reference Signs List

1 Main body of magnetic treatment apparatus
2 Casing
3 Display (touch input-type display)
11 Probe of magnetic treatment apparatus
12 Outer package
13 Circuit part
14 Flexible sheet (printed board)
15 Coil for high-frequency output
16 Coil for magnetic field intensity detection
17 Coil for low-frequency output
18 Slit section
19 Bridge portion
21 Signal cable
31 Copper foil
32 Skin portion
33 Upper surface
34 Lower surface

**Claims**

1. A magnetic treatment apparatus for treating pain in an affected area of a living body by producing a biostimulation signal wave and stimulating the cells and nerves in and around the affected area as a result of irradiating the affected area of the living body with a magnetic field for stimulating the affected area that is generated at a coil via the biostimulation signal wave, comprising:

an apparatus main body having a signal wave output part configured to produce and output a biostimulation high-frequency signal; and

a probe formed separately from the apparatus main body and having a coil for high-frequency output that is connected to the signal wave output part through a signal cable and is supplied with the biostimulation high-frequency signal output from the signal wave output part,

**characterized in that**

a high-frequency current of 100 MHz or higher is applied to the coil for high-frequency output, the coil for high-frequency output is formed as a planar body on both surfaces of an insulating flexible sheet, and centers of magnetic fields generated by the coils on both surfaces are coincident with each other.

2. The magnetic treatment apparatus according to claim 1, wherein the coil for high-frequency output is made of a conductor having a thickness that is not smaller than twice a current skin depth $d_i$ calculated from the frequency of the high-frequency current, and is not larger than 70 $\mu$m.

3. The magnetic treatment apparatus according to claim 2, wherein the conductor is a metal foil.

4. The magnetic treatment apparatus according to any one of claims 1 to 3, wherein

the signal wave output part also produces and outputs a biostimulation low-frequency signal, and

the probe also has a coil for low-frequency output that is connected to the signal wave output part through the signal cable and is supplied with the biostimulation low-frequency signal output from the signal wave output part.

Fig. 1

Fig. 2(a)

11

12

13

A

A

21

Fig. 2(b)

11

21

Fig. 2(c)

14

11

21

Fig. 3(a)

Fig. 3(b)

Fig. 4

# INTERNATIONAL SEARCH REPORT

| | International application No. |
|---|---|
| | **PCT/JP2023/000402** |

**A. CLASSIFICATION OF SUBJECT MATTER**

***A61N 2/02***(2006.01)i
FI: A61N2/02 A

According to International Patent Classification (IPC) or to both national classification and IPC

**B. FIELDS SEARCHED**

Minimum documentation searched (classification system followed by classification symbols)

A61N2/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2021-112568 A (CHIU, Hsuanhua) 05 August 2021 (2021-08-05)<br>paragraphs [0001], [0025]-[0036], fig. 9-11 | 1-4 |
| Y | JP 2019-5142 A (NIPRO CORPORATION) 17 January 2019 (2019-01-17)<br>paragraphs [0021], [0028], [0040]-[0042], [0067] | 1-4 |
| Y | WO 2006/115119 A1 (THOSHIN CO., LTD.) 02 November 2006 (2006-11-02)<br>paragraphs [0027]-[0029], [0036]-[0038], fig. 6 | 2-3 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 March 2023** | **28 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/000402**

| Patent document cited in search report | | | Publication date (day/month/year) | Patent family member(s) | | | Publication date (day/month/year) |
|---|---|---|---|---|---|---|---|
| JP | 2021-112568 | A | 05 August 2021 | TW | 202128248 | A | |
| JP | 2019-5142 | A | 17 January 2019 | US | 2020/0197717 | A1 | |
| | | | | paragraphs [0050], [0057], [0069]-[0071], [0096] | | | |
| | | | | WO | 2018/235629 | A1 | |
| | | | | EP | 3643359 | A1 | |
| | | | | CN | 110785207 | A | |
| WO | 2006/115119 | A1 | 02 November 2006 | JP | 2006-296668 | A | |
| | | | | TW | 200640527 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008056414 A
- JP S631988160676 A
- JP S631988160677 A